Europäisches Patentamt

European Patent Office

Office européen des brevets

(11) Veröffentlichungsnummer: **0 158 333**
**B1**

# (12) EUROPÄISCHE PATENTSCHRIFT

(45) Veröffentlichungstag der Patentschrift:
12.10.88

(21) Anmeldenummer: 85104300.0

(22) Anmeldetag: 09.04.85

(51) Int. Cl.⁴: **A 61 C 8/00**

(54) **Enossales Implantat zur Befestigung von festsitzendem Zahnersatz.**

(30) Priorität: 11.04.84 DE 3413578

(43) Veröffentlichungstag der Anmeldung:
16.10.85 Patentblatt 85/42

(45) Bekanntmachung des Hinweises auf die Patenterteilung:
12.10.88 Patentblatt 88/41

(84) Benannte Vertragsstaaten:
AT BE CH FR GB IT LI LU NL SE

(56) Entgegenhaltungen:
EP-A-0 015 599
DE-C-2 413 883

TOOLING & PRODUCTION, Band 47, Nr. 1, April
1981, Seiten 92-96, Solon, Ohio, US; "Deep cold
puts metal on the move"

(73) Patentinhaber: IMPLANTO- LOcK Gesellschaft mit
beschränkter Haftung für Implantatforschung
und Entwicklung, Ness 1, D-2000 Hamburg 11 (DE)

(72) Erfinder: **Koch, Werner Lutz, Lönsweg 7, D-3073
Liebenau (DE)**

(74) Vertreter: **Richter, Werdermann & Gerbaulet,
Neuer Wall 10, D-2000 Hamburg 36 (DE)**

EP 0 158 333 B1

## Beschreibung

Die Erfindung betrifft ein enossales Implantat zur Befestigung von festsitzendem Zahnersatz der im Oberbegriff des Anspruchs 1 genannten Art.

Durch die DE-C-2 413 883 ist ein enossales Implantat der betreffenden Art bekannt, bei dem in die als Gewindebohrung ausgebildete Bohrung des in den Kieferknochen einbringbaren Teils ein Gewindefutter mit Außengewinde aus nachgiebigem Material auswechselbar eingeschraubt ist, das eine Gewindebohrung aufweist, in die ein Gewindezapfen des anderen, zur Befestigung des festsitzenden Zahnersatz dienenden Gewindeteils eingeschraubt ist, derart, daß sich die beiden Teile nicht berühren. Durch diese elastische Zwischenlage in Form eines Gewindefutters soll der Zahnhalteapparat eines natürlichen Zahnes imitiert werden. Durch die Verschraubbarkeit ist ein Auswechseln und Abstimmen der Nachgiebigkeit des Gewindefutters in bezug auf die gewünschte imitierende Wirkung möglich.

Dieses bekannte Implantat erfüllt gestellte Forderungen hinsichtlich Imitation des Zahnhalteapparates und der Abstimmbarkeit voll und ganz, jedoch hat sich gezeigt, daß die Verschraubungsflächen des Gewindefutters zu Infektionen führen können. Die Ursache hierfür liegt darin, daß sich die Verschraubungskräfte verringern und Spalte entstehen können, in denen sich Bakterien festsetzen oder bilden können, die zu Entzündungen führen.

Der Erfindung liegt die Aufgabe zugrunde, ein enossales Implantat der betreffenden Art zu schaffen, bei dem keine Lockerung auftreten kann und sich somit auch keine Bakterienherde bilden und Entzündungen entstehen können.

Die der Erfindung zugrundeliegende Aufgabe wird durch die im Kennzeichen des Anspruchs 1 angegebene Lehre gelöst.

Die Erfindung geht von dem Prinzip der Verschraubung ab und sieht statt dessen eine Klemmverbindung zwischen einem zapfen und einer entsprechenden Bohrung vor. Die Bemessung der Durchmesser ist dabei in Verbindung mit der Wahl der Wärmeausdehnungszahl so bemessen, daß bei normaler Körpertemperatur ein ausreichender Klemmsitz erzielt ist. Gleichzeitig ist die Bemessung so getroffen, daß bei Temperaturabsenkung erreicht werden kann, daß der Durchmesser des Zapfens kleiner wird und somit ein Herausziehen des Zapfens aus der Bohrung möglich ist.

Die Verkleinerung des Zapfens durch Temperaturabsenkung ist dann, wenn für Zapfen und das die Bohrung aufweisende Teil ein Material verwendet ist, das eine gleiche Wärmeausdehnungszahl hat, dadurch möglich, daß nur der Zapfen abgekühlt wird. Dabei muß eine schnelle Abkühlung erfolgen, um eine gleichzeitige Abkühlung des die Bohrung aufweisenden Materials zu vermeiden. Eine solche schnelle Abkühlung ist jedoch durchaus mit Flüssigkeiten möglich, die bei Körpertemperatur verdampfen.

Gemäß einer Weiterbildung der Grundlehre besteht der Zapfen aus einem Material, dessen Wärmeausdehnungszahl größer als die des die Bohrung aufweisenden Materials ist. In diesem Fall ist sichergestellt, daß auch dann, wenn das die Bohrung aufweisende Teil die Abkühlung mitmacht, wegen der unterschiedlichen Wärmeausdehnungszahl sich die Durchmesserdifferenz ändert und so ein Herausziehen des Zapfens aus der Bohrung möglich macht.

Als Material für den Zapfen und/oder das die Bohrung aufweisende Teil eignet sich besonders gut Metall, jedoch ist auch ein Nichtmetall, insbesondere Kunststoff, in gleicher Weise geeignet. Dies insbesondere für den Zapfen, so daß trotz der bei Kunststoff an sich bestehenden, eingangs beim Stand der Technik geschilderten Nachteile der Einsatz von Kunststoff möglich ist.

Von besonderem Vorteil bei der Erfindung ist die Tatsache, daß die Verbindungskräfte im wesentlichen quer zur normalen Belastung des Implantats durch Kaukräfte wirken. Während Kaukräfte bei einem herkömmlichen Implantat die durch die Verschraubung bewirkten Zugkräfte und damit die Haltekräfte verringern, lassen sie die Haltekräfte bei der erindungsgemäßen Lösung unbeeinträchtigt.

Aus Gründen der Halterung des Zapfens ist es zweckmäßig, diesen möglichst lang zu machen. Andererseits erhöht jedoch eine große Länge des Zapfens eine Abkühlung desselben bis zu seinem Ende hin. Zur Überwindung dieser Schwierigkeit dient die Lehre des Anspruchs 4. Die bei Körpertemperatur mit Schiebesitzpassung gehaltene Verlängerung des Zapfens trägt wesentlich zu einer guten Halterung des Zapfens bei, wegen des Wärmewiderstandes zwischen Zapfen und Verlängerung wird ein Kälteabfluß zu der Verlängerung hin vermieden, so daß die Temperatur des Zapfens schnell und stark absenkbar und so ein sicheres Lösen des Zapfens möglich ist. Die Ausführung dieses Gedankens kann sogar so weit gehen, daß der Zapfen selbst nur noch als Element dient, das das Gesamtgebilde, bestehend aus Zapfen und Verlängerung, in der entsprechenden Bohrung des in den Kieferknochen eindringbaren Teils in Axialrichtung hält, während die wesentlichen übrigen Kräfte von der Verlängerung in das genannte Teil übertragen werden. In Verfolg dieses Grundgedankens ist es sogar auch möglich, den Zapfen aus Halterungsgründen zwar sehr lang zu gestalten, die erfindungsgemäße Dimensionierung der Durchmesser jedoch nur im Bereich der Wurzel des Zapfens vorzusehen, in dem eine Abkühlung keine Schwierigkeiten bereitet.

Die Bohrung, in der die Verlängerung des Zapfens sitzt, hat zweckmäßigerweise den gleichen Durchmesser wie die Bohrung für den Zapfen, sie kann jedoch auch kleiner sein, wozu es natürlich eines zusätzlichen Arbeitsganges bedarf.

Die Lehre des Anspruchs 6 betrifft die Ausbildung des wärmewiderstandes zwischen Zapfen und Verlängerung. Er hat die Form einer Buchse, die in einer Bohrung des Zapfens sitzt, wobei die Verbindung zwischen Zapfen und Verlängerung durch einen Stift gebildet ist, der sich an der Verlängerung befindet und in die Buchse eingreift. Der Stift kann gemäß der Lehre des Anspruchs 7 auf der anderen Seite der Buchse herausragen und dort Mittel zur Befestigung des festsitzenden Zahnersatzes aufweisen. Auf diese Weise wird ein großer Teil der vom Zahnersatz in den Knochen einzuleitenden Kräfte durch den Stift in das im Knochen sitzende Teil des Implantats eingeleitet. Die Buchse weist an ihren Enden zweckmäßigerweise axial nach außen gerichtete Flansche auf, die die Stirnkanten des Zapfens bzw. des Teils hintergreifen. Statt der Flansche können auch einzelne Scheiben zwischengelegt sein.

Nach der Lehre des Anspruchs 7 erstreckt sich die Buchse bis in das Teil hinein, das im Kieferknochen zu verankern ist. Dabei erstreckt sich der Stift in diese Verlängerung der Buchse, er ist nicht unmittelbar mit dem im Kieferknochen zu verankernden Teil des Implantats verbunden und damit von diesem auch wärmemäßig isoliert. Bei Abkühlung des zur Befestigung des Zahnersatzes dienenden Teils bzw. des daraus vorstehenden Stiftes ist auf diese Weise eine Ableitung der Kälte durch den Stift in das im Kieferknochen sitzende Implantatteil vermieden.

Nach der Lehre des Anspruchs 10 ist der Stift mittels eines an seinem Ende vorgesehenen Gewindezapfens mit der Verlängerung der Buchse verschraubt. Nach Anspruch 11 ist auch die Buchse mittels eines an ihrem Ende vorgesehenen Gewindezapfens mit dem im Kieferknochen sitzenden Implantatteil verschraubt. Diese Maßnahmen bilden alle zusammen mit dem zur Befestigung des Zahnersatzes dienenden Teil herausnehmbaren Teile eine Einheit.

Nach der Lehre des Anspruchs 12 hat die Verlängerung des Zapfens an dem zur Befestigung des Zahnersatzes dienenden Teil einen höheren Elastizitätsmodul als der Zapfen. Dadurch werden Spannungsspitzen reduziert und das Verschieben der Verlängerung in der zugehörigen Bohrung erleichtert.

Weitere Einzelheiten und Vorteile der Erfindung sollen nachfolgend anhand der Zeichnung näher erläutert werden.

Fig. 1 ist ein Teilschnitt durch ein Ausführungsbeispiel eines enossalen Implantats gemäß der Erfindung,

Fig. 2 zeigt eine Abwandlung des Ausführungsbeispiels gemäß Fig. 1.

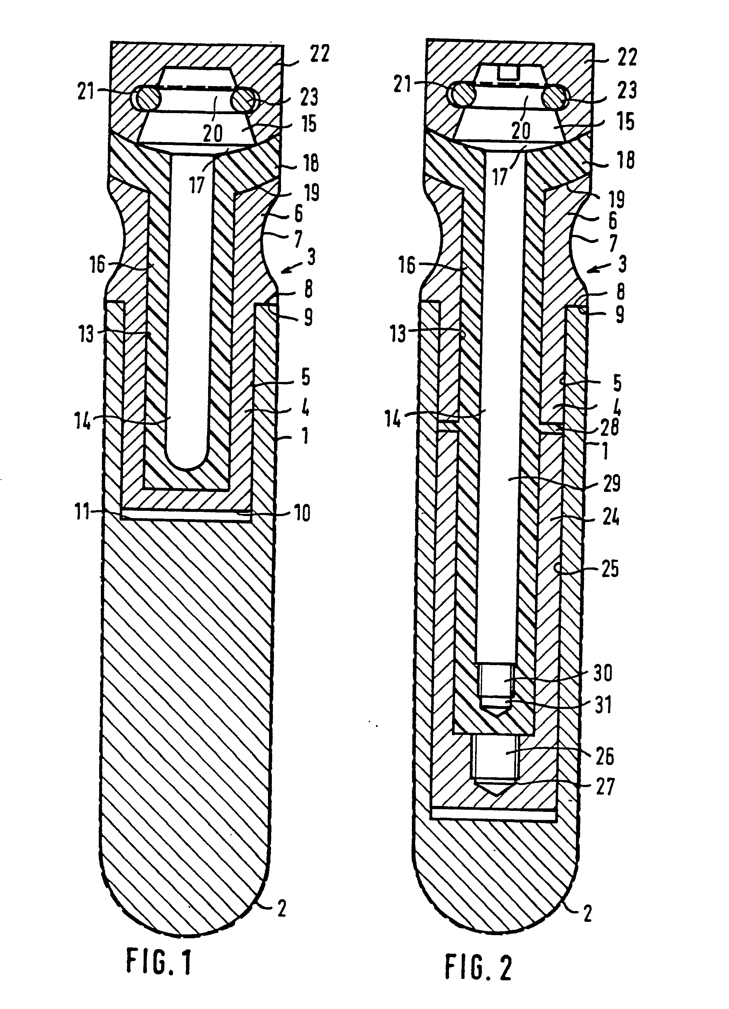

Das in Fig. 1 dargestellte Implantat weist ein Teil 1 auf, das ein halbkugelförmiges Ende 2 hat, in einen Kieferknochen einbringbar ist, sowie ein anderes Teil 3, das mit einem zylindrischen Zapfen 4 mit Klemmsitz in eine Bohrung 5 eingreift. An seinem oberen Ende weist das andere Teil 3 einen Kopf 6 auf, in dem sich eine seitliche Einschnürung 7 befindet, die bei eingesetztem Implantat in der Höhe des Zahnfleisches bzw. des Austritts daraus liegt. Der Kopf 6 bildet auf seiner Unterseite eine Aschlagkante 8, die gegen eine Oberkante 9 des einen Teils 1 stößt. Ein unteres Ende 10 des Zapfens 4 endet kurz vor einem Boden 11 der Bohrung 5.

Der Zapfen 4 besteht aus einem Material, dessen Wärmeausdehnungszahl größer als die des Materials des Teils 1 ist, und hat einen Durchmesser, der im nicht eingesetzten Zustand bei Körpertemperatur soviel größer als der Durchmesser der Bohrung ist, daß ein fester Klemmsitz geschaffen ist.

In dem anderen Teil 3 bzw. in Zapfen 4 und Kopf 6 befindet sich eine Bohrung 13, in die ein als zylindrischer Zapfen 14 ausgebildeter Fortsatz an einem Konus 15 so eingreift, daß ein Zwischenraum gebildet ist, in dem eine Zwischenlage 16 aus nachgiebigem Material, z. B. Kunststoff, angeordnet ist. Die Unterseite des Konus 15 bildet eine Auflagefläche 17, die auf einem flanschartigen Teil 18 der Zwischenlage 16 aufliegt. Der flanschartige Teil liegt auf seiner Unterseite auf einer Auflagefläche 19 am oberen Ende des Kopfes 6 auf. Die Auflagefläche 17 und 19 sind kalottenförmig. Sämtliche Flächen der Zwischenlage 16 bzw. des flanschartigen Teils aus nachgiebigem Material, die mit Flächen benachbarter Teile in Kontakt stehen, haften fest an diesen, sind also z. B. verklebt oder vergossen.

In der äußeren konischen Fläche des Konus 15 befindet sich eine umlaufende Rille 20, der radial gegenüber sich eine Rille 21 in der komplementären inneren konischen Paßfläche eines Befestigungsteils 22 befindet. In diese Rillen 20 und 21 greift eine im wesentlichen U-förmige Haltefeder 23 ein, die so beide Teile 15 und 22 fest miteinander verbindet. Zum Einsetzen der U-förmigen Haltefeder 23 ist in dem Befestigungsteil 22 ein seitlicher Schlitz vorgesehen. Diese Verbindung ist spielfrei und leicht lösbar. Darüber hinaus werden Torsionskräfte nicht übertragen so daß auch das andere Teil des Pfostens nicht mit diesen Kräften belastet wird und sich daher nicht lösen kann.

Bei Verwendung des dargestellten Implantats wird zunächst in einen Kieferknochen eine zu dem einen Teil 1 im wesentlichen komplementäre Ausnehmung gefräst und das Teil 1 eingesetzt. Dabei ist die Bohrung 5 durch ein dem anderen Teil 3 ähnliches, jedoch nur einen kurzen Kopf aufweisendes Teil geschlossen, über dem auch das Zahnfleisch geschlossen wird, so daß das Teil 1 fest einheilen kann. Danach wird das Zahnfleisch über dem Teil 1 perforiert und der Verschluß der Bohrung 5 entfernt. Das andere Teil 3 wird abgekühlt und dann schnell mit seinem Zapfen 4 in die Bohrung 5 eingesetzt.

Sobald die Temperatur des Zapfens 4 auf die Körpertemperatur angestiegen ist, ist eine feste Klemmverbindung zwischen den beiden Teilen 1 und 3 des Implantats gegeben. Dabei sind die mechanischen Eigenschaften der nachgiebigen Zwischenlage 16 bzw. des flanschartigen Teils 18 entsprechend den gewünschten zu imitierenden Werten des Zahnhalteapparates ausgewählt. Ist das Teil 3 so befestigt, so steht der Konus 15 frei aus dem Zahnfleisch vor, so daß darauf das Befestigungsteil 22 gesetzt werden kann, das inzwischen mit einem Zahnersatz verbunden worden ist. Die Befestigung des Befestigungsteils 22 mit dem daran sitzenden Zahnersatz erfolgt durch seitliches Einschieben der Haltefeder 23, die jederzeit zum Zwecke von Inspektionen oder zur Reinigung herausgenommen werden kann, um so den Zahnersatz abnehmen zu können.

Soll das Teil 3 wieder entfernt werden, so wird der Kopf 6 des Teils 3 beispielsweise durch Aussprühen mit einer schnell verdampfenden Flüssigkeit gekühlt, wodurch auch der Zapfen 4 gekühlt wird, dessen Durchmesser sich dadurch so verkleinert, daß sich der Zapfen 4 aus der Bohrung 5 herausziehen läßt.

Fig. 2 zeigt eine Abwandlung der Ausführungsform gemäß Fig. 1. Sich entsprechende Teile sind mit gleichen Bezugsziffern bezeichnet. Der unterschied besteht darin, daß eine Verlängerung 24 vorgesehen ist, die sich in der entsprechend verlängerten Bohrung 5 mit Schiebesitz bei Körpertemperatur erstreckt. Die als Buchse ausgebildete Zwischenlage 16 erstreckt sich in eine Bohrung 25 der Verlängerung 24 und ist mittels eines Gewindezapfens 26 in einem Gewindesackloch 27 der Verlängerung 24 verschraubt. zwischen den gegenüberliegenden Stirnflächen des Zapfens 4 und der Verlängerung 24 erstreckt sich ein Flansch 28 der die Buchse bildenden Zwischenlage 16, die aus schlecht wärmeleitendem Material, z. B. Kunststoff, besteht und so beide Teile gegenüber Wärmeleitung isoliert.

Der Zapfen 14 ist zu einem Stift 29 verlängert, der mittels eines Gewindezapfens 30 in einem Gewindesackloch 31 verschraubt ist.

Die Teile 14, 29 bis 31, 18, 16, 26, 27, 3, 4 und 24 bilden somit eine Einheit, die gemeinsam in die Bohrung 5 des Teils 1 einsetzbar ist, wobei hinsichtlich der Kühlung des Zapfens 4 die entsprechenden Ausführungen in Verbindung mit Fig. 1 gelten.

Der Grundgedanke der Erfindung, zwei Teile von Implantaten durch Wärmedehnung bzw. -schrumpfung zu verbinden, ist natürlich auch bei der Verbindung zwischen dem zur Befestigung des Zahnersatzes dienenden Teil und dem Zahnersatz selbst anwendbar. Er ist sogar auch bei Verbindung von Teilen des Zahnersatzes selbst anwendbar.

**Patentansprüche**

1. Enossales Implantat zur Befestigung von festsitzendem Zahnersatz, das zwei miteinander verbindbare Teile (1, 3) aufweist, von denen das eine Teil (1) in einen Kieferknochen einbringbar ist und eine axiale Bohrung (5) aufweist, in die ein Zapfen (4) des anderen, zur Befestigung des Zahnersatzes dienenden Teils (3) auswechselbar einbringbar ist, dacurch gekennzeichnet, daß der Durchmesser der zylindrischen Außenwandung des Zapfens (4) soviel größer als der Durchmesser der Bohrung (5) bemessen ist, daß der Zapfen (4) bei Körpertemperatur in der Bohrung (5) klemmt, bei Temperaturabsenkung niedriger als die Körpertemperatur jedoch aus der Bohrung (5) herausziehbar ist.

2. Enossales Implantat nach Anspruch 1, dadurch gekennzeichnet, daß der Zapfen (4) aus einem Material besteht, dessen Wärmeausdehnungszahl größer als die des die Bohrung (5) aufweisenden Materials ist.

3. Enossales Implantat nach Anspruch 1, dadurch gekennzeichnet, daß das Material des Zapfens (4) und/oder die Bohrung (5) aufweisenden Teils (1) Metall oder Nichtmetall, insbesondere Kunststoff, ist.

4. Enossales Implantat nach Anspruch 1, dadurch gekennzeichnet, daß der Zapfen (4) eine Verlängerung (24) aufweist, daß sich zwischen dem Zapfen (4) und der Verlängerung (24) Material mit geringer Wärmeleitfähigkeit befindet und daß die Verlängerung (24) in einer Bohrung (25) mit solcher Passung sitzt, daß die Verlängerung (24) bei Körpertemperatur in der Bohrung (25) verschiebbar ist.

5. Enossales Implantat nach Anspruch 4, dadurch gekennzeichnet, daß die Bohrung (25), in der die Verlängerung (24) des Zapfens (4) sitzt, den gleichen Durchmesser hat wie die Bohrung (5) für den Zapfen (4).

6. Enossales Implantat nach Anspruch 4, dadurch gekennzeichnet, daß der Zapfen (4) eine axiale Bohrung aufweist, daß das Material mit geringer Wärmeleitfähigkeit eine Buchse (16) bildet, die in einer axialen Bohrung (13) des Zapfens (4) sitzt, in die ein Stift (29) eingreift.

7. Enossales Implantat nach Anspruch 6, dadurch gekennzeichnet, daß sich die Bohrung (13) im Zapfen (4), die Buchse (16) und der Stift (29) durch den Zapfen (4) und durch das zur Befestigung des Zahnersatzes dienende Teil (3) hindurcherstrecken, wobei der Stift (29) auf der der Verlängerung (24) abgewandten Seite vorsteht und Mittel (15) zur Befestigung des festsitzenden Zahnersatzes aufweist.

8. Enossales Implantat nach Anspruch 7, dadurch gekennzeichnet, daß die Buchse (16) an ihren der Verlängerung (24) abgewandten Enden sich nach außen erstreckende Flansche (28, 18) aufweist, deren Durchmesser höchstens so groß ist wie der des benachbarten zur Befestigung des Zahnersatzes dienenden Teils (3) bzw. des Zapfens (4).

9. Enossales Implantat nach Anspruch 8,

dadurch gekennzeichnet, daß statt der Flansche (28) einzelne Scheiben vorgesehen sind.

10. Enossales Implantat nach Anspruch 6, dadurch gekennzeichnet, daß die Buchse (16) eine Verlängerung aufweist, die sich in eine Bohrung der Verlängerung (24) des Zapfens (4) an dem zur Befestigung des Zahnersatzes dienenden Teil (3) erstreckt und daß der Stift (29) in der Verlängerung der Buchse (16) steckt.

11. Enossales Implantat nach Anspruch 10, dadurch gekennzeichnet, daß der Stift (29) mittels eines Gewindezapfens (30) in einem Gewindesackloch (31) in der Verlängerung der Buchse (16) befestigt ist.

12. Enossales Implantat nach Anspruch 10, dadurch gekennzeichnet, daß die Buchse (16) mittels eines Gewindezapfens (26) in einem Gewindesackloch (27) in der Verlängerung (24) des Zapfens (4) des zur Befestigung des Zahnersatzes dienenden Teils (3) befestigt ist.

13. Enossales Implantat nach Anspruch 4, dadurch gekennzeichnet, daß die Verlängerung (24) des Zapfens (4) an dem zur Befestigung des Zahnersatzes dienenden Teil (3) einen höheren Elastizitätsmodul hat als der Zapfen.

## Claims

1. Endosseous implant for the attachment of a fixed dental prothesis, consisting of two parts (1, 3) which can be attached to each other, one part of which (1) being implantable into a jaw bone and having an axial bore hole (5) into which a peg (4) of the other part serving as a means of attachment of the dental prothesis can be inserted, and being exchangeable, characterized in that the diameter of the cylindrical outer wall of the peg (4) is larger than the diameter of the bore hole (5) to such an extent that the peg (4) clamps in the bore hole (5) at body temperature, but that it can be removed from the bore hole (5) when the body temperature falls below the normal body temperature.

2. Endosseous implant according to claim 1, characterized in that the peg (4) consists of a material, of which the coefficient of thermal expansion is higher than the coefficient of the material with the bore hole (5).

3. Endosseous implant according to claim 1, characterized in that the material of the peg (4) and/or the part with the bore hole (5) is of metal or non metallic material, especially plastic material.

4. Endosseous implant according to claim 1, characterized in that the peg (4) has an extension (24) so that material with a low coefficient of thermal expansion is situated between the peg (4) and the extension (24) and that the extension (24) fits into a bore hole (25) in such a manner that the extension (24) can be moved in the bore hole (25) at body temperature.

5. Endosseous implant according to claim 4, characterized in that the diameter of the bore hole (25) with the implanted extension (24) of the peg (4) is the same as that of the bore hole (5) for the peg (4).

6. Endosseous implant according to claim 4, characterized in that the peg (4) has an axial bore hole, that the material with low thermal conductivity forms a bearing bush (16) which is located in an axial bore hole (13) of the peg (4) into which a pin (29) catches.

7. Endosseous implant according to claim 6, characterized in that the bore hole (13) in the peg (4), the bush (16) and the pin (29) extend through the peg (4) and through the part (3) serving as the means of attachment of the dental prothesis, whereby the pin (29) protrudes on the side turned away from the extension (24) and has means (15) for the attachment of the fixed dental prothesis.

8. Endosseous implant according to claim 7, characterized in that the bush (16) at the ends turned away from the extension (24) has flanges (28, 18) extending outwardly, the diameter of which is at the most equal to that of the adjacent part (3) serving as the means of attachment of the dental prothesis or of the peg (4).

9. Endosseous implant according to claim 8, characterized in that individual disks are provided instead of the flanges (28).

10. Endosseous implant according to claim 6, characterized in that the bush (16) has an extension extending into a bore hole of the extension (24) of the peg (4) at the part (3) serving as the means of attachment of the dental prothesis and that the pin (29) is inserted into the extension of the bush (16).

11. Endosseous implant according to claim 10, characterized in that the pin (29) is attached by means of a threaded stem (30) in a threaded blind hole (31) in the extension of the bush (16).

12. Endosseous implant according to claim 10, characterized in that the bush (16) is attached by means of a threaded stem (26) in a threaded blind hole (27) in the extension (24) of the peg (4) of the part (3) serving as a means of attachment of the dental prothesis.

13. Endosseous implant according to claim 4, characterized in that the extension (24) of the peg (4) at the part (3) serving as the means of attachment of the dental prothesis has a larger elastic modulus than the peg.

## Revendications

1. Implant endo-osseux pour la fixation d'une superstructure dentaire, qui présente deux pièces (1, 3) pouvant être reliées l'une à l'autre, l'une d'entre elles (1) pouvant être mise en place dans un maxillaire et présentant une forure axiale (5), dans laquelle un tourillon (4) de l'autre pièce (3), qui sert à la fixation de la superstructure dentaire, peut être mis en place de manière à être remplaçable, caractérisé en ce que le diamètre de la paroi cylindrique extérieure du tourillon (4) est dimensionné de manière à être d'autant plus grand que le diamètre de la forure (5) de telle sorte que le tourillon (4) se bloque dans la forure (5), à la température du corps, tout en pouvant être cependant retiré de la forure (5), en cas de chute de la température à une température inférieure à la température du corps.

2. Implant endo-osseux selon la revendication 1, caractérisé en ce que le tourillon (4) est en une matière dont le coefficient de dilatation thermique est supérieur à celui présenté par la matière de la forure (5).

3. Implant endo-osseux selon la revendication 1, caractérisé en ce que la matière du tourillon (4) et/ou de la pièce (1) qui présente la forure (5) est du métal ou du non-métal, en particulier une matière synthétique.

4. Implant endo-osseux selon la revendication 1, caractérisé en ce que le tourillon (4) présente un prolongement (24), qu'il se trouve de la matière à faible conductibilité thermique entre le tourillon (4) et le prolongement (24) et que le prolongement (24) est calé dans une forure (25) avec un ajustement tel que le prolongement (24) est mobile dans la forure (25), à la température du corps.

5. Implant endo-osseux selon la revendication 4, caractérisé en ce que la forure (25), dans laquelle le prolongement (24) du tourillon (4) est calé, a le même diamètre que la forure (5) du tourillon (4).

6. Implant endo-osseux selon la revendication 4, caractérisé en ce que le tourillon (4) présente une forure axiale, que la matière à faible conductibilité thermique forme une douille (16) qui est calée dans une forure axiale (13) du tourillon (4), dans laquelle s'engrène une broche (29).

7. Implant endo-osseux selon la revendication 6, caractérisé en ce que la forure (13) du tourillon (4), la douille (16) et la broche (29) s'étendent à travers le tourillon (4) et la pièce (3) qui sert à la fixation de la superstructure dentaire, la broche (29) faisant saillie sur le côté opposé au prolongement (24) et présentant des moyens (15) de fixation de la superstructure dentaire.

8. Implant endo-osseux selon la revendication 7, caractérisé en ce que la douille (16) présente, à ses extrémités opposées au prolongement (24), des brides (28, 18) qui s'étendent vers l'extérieur, dont le diamètre est au maximum aussi grand que celui de la pièce (3) voisine, qui sert à la fixation de la superstructure dentaire, ou du tourillon (4).

9. Implant endo-osseux selon la revendication 8, caractérisé en ce que des rondelles isolées sont prévues à la place des brides (28).

10. Implant endo-osseux selon la revendication 6, caractérisé en ce que la douille (16) présente un prolongement qui s'étend dans une forure du prolongement (24) du tourillon (4) sur la pièce (3) qui sert à la fixation de la superstructure dentaire et que la broche (29) est fixée dans le prolongement de la douille (16).

11. Implant endo-osseux selon la revendication 10, caractérisé en ce que la broche (29) est fixée au prolongement de la douille (16) à l'aide d'une goupille filetée (30) dans un trou borgne fileté (31).

12. Implant endo-osseux selon la revendication 10, caractérisé en ce que la douille (16) est fixée au prolongement (24) du tourillon (4) de la pièce (3) qui sert à la fixation de la superstructure dentaire à l'aide d'une goupille filetée (26) dans un trou borgne fileté (27).

13. Implant endo-osseux selon la revendication 4, caractérisé en ce que le prolongement (24) du tourillon (4) de la pièce (3) qui sert à la fixation de la superstructure dentaire a un module d'élasticité supérieur à celui du tourillon.

FIG. 1

FIG. 2